# NEW EUROPEAN PATENT SPECIFICATION

(11) **EP 0 506 757 B2**
(45) Date of publication and mention of the opposition decision: **26.10.2005**
(45) Mention of the grant of the patent: 26.08.1998
(21) Application number: 91901327.6
(22) Date of filing: 06.12.1990
(51) Int. Cl.: C12N 15/12, C07K 14/00, C12P 21/02

(54) **A RECOMBINANT HUMAN FACTOR VIII DERIVATIVE**
REKOMBINANTER ABKÖMMLING DES MENSCHLICHEN FAKTORS III
DERIVE DU FACTEUR HUMAIN VIII DE RECOMBINAISON

(30) Priority: 15.12.1989 SE 8904239
(43) Date of publication of application: 07.10.1992
(60) Divisional application: 97103685.0
(73) Proprietor: BIOVITRUM AB, 112 76 Stockholm (SE)
(72) Inventor: ALMSTEDT, Anneli, B., S-113 29 Stockholm (SE); HELLSTRÖM, Eva, Maria, S-112 23 Stockholm (SE); LARSSON, Kerstin, S-196 35 Kungsängen (SE); LIND, Peter, S-752 27 Uppsala (SE); SANDBERG, Helena, Inga, S-161 37 Bromma (SE); SPIRA, Jack, S-171 35 Stockholm (SE); SYDOW-BÄCKMAN, Mona, S-117 24 Stockholm (SE)
(74) Representative: Pohlman, Sandra M.
(86) International application number: PCT/SE1990/000809
(87) International publication number: WO 1991/009122

(56) References cited:
- EP-A- 0 251 843
- EP-A- 0 303 540
- Biochemistry, Vol. 25, No. 26, 1986; DAN L. EATON et al: "Construction and Characterization of an Active Factor VIII Variant Lacking the Central One-Third of the Molecule", see page 8343 - page 8347.
- The Journal of Cell Biology, Vol. 105, No. 6, 1987; ANDREW J. DORNER et al: "The Relationship of N-linked Glycosylation and Heavy Chain-binding Prote in Association with the Secretion of Glycoproteins", see page 2665 - page 2674.

## Description

### A recombinant human factor VIII derivative

The present invention relates to a DNA sequence coding for a biologically active recombinant human factor VIII derivative, a recombinant expression vector containing such DNA sequence, host cells transformed with such recombinant expression vector and a process for the manufacture of the recombinant human factor VIII derivative. The invention also covers the human factor VIII derivative comprising two polypeptides linked by a metal ion bridge.

### BACKGROUND OF THE INVENTION

Classic hemophilia or hemophilia A is the most common of the inherited bleeding disorders. It is a result of a chromosome X-linked deficiency in blood coagulation factor VIII. It affects almost exclusively males and the incidence is one to two individuals per 10 000 men. The X-chromosome defect is transmitted by female carriers who are not themselves hemophiliacs. Depending on the type of mutation in the X-chromosome, factor VIII is either dysfunctional or absent which leads to a retarded blood coagulation. The clinical manifestation of hemophilia A Is an abnormal bleeding tendency and before treatment with factor VIII concentrates was introduced the mean life time for a person with severe hemophilia A was less than 20 years. The use of concentrates of factor VIII from plasma has considerably improved the situation for the hemophilia patients. The mean life time has increased extensively and most of them have the possibility to live a more or less normal life. However, there have been certain problems with the concentrates and their use. The most serious have been the transmission of viruses. Hitherto, viruses causing AIDS, hepatitis B, and non A non B hepatitis have hit the hemophiliac population seriously. Recently, different virus inactivation methods have been introduced in the production process for many commercial concentrates. There are also new highly purified factor VIII concentrates coming out on the market which are expected to be much safer in comparison to older concentrates as regards the risk for virus transmission. However, no guarantees on the absence of virus can be made. Furthermore, the concentrates are fairly expensive because the raw material plasma is expensive due to linked supple

A recombinant factor VIII is likely to solve a large extent of the problems associated with the use of plasma derived factor VIII concentrates. In view of the need of a recombinant factor VIII for the treatment of hemophilia A, a couple of groups are presently working on the development of such a product. However, the development of a recombinant factor VIII has met some difficulties. One of the major problems is to produce recombinant factor VIII in sufficiently high yields. Most of the development work done has been performed with use of the cDNA gene coding for the full-length factor VIII molecule.

This invention describes a dalsted factor VIII cDNA which codes for a recombinant factor VIII derivative, corresponding, as regards to molecular weight and other biochemical characteristics, to a previously described plasma factor VIII form present in considerable amounts in commercial concentrates (Anderson, L-O. et al (1986), Proc.Natl. Acad.ScL USA 83, 2979-2983). The expression level obtained upon expressing this molecule in an animal cell culture has proven to be considerably higher as compared to when the full-length factor VIII cDNA is used to express factor VIII. The new deleted factor VIII cDNA is likely to give sufficiently high yields of recombinant factor VIII to be used in an industrial process for a pharmaceutical preparation of recombinant factor VIII.

### DEFINITIONS USED

In the following sections, the nomenclature of the different structural domains of the 2332 amino acid single stranded full-length factor VIII is similar to those defined by Vehar, GA. et al (1984), Nature 312, 337-342. Accordingly, the A1 and A2 domains are contained within the factor VIII heavy chain form of approximately 200 kDa (amino acids 1 through 1648) to approximately 90 kDa (amino acids 1 through 740). The A3, C1 and C2 domains are contained within the factor VIII light chain of approximately 80 kDa (amino acids 1649 through 2332). The B domain constitutes the heavily glycosylated middle region of full-length single chain factor VIII or the C-terminal part of the 200 kDa form of the factor VIII heavy chain (amino acids 741 through 1648). The term "factor VIII deletion derivative" is defined as one or more polypeptide chains having factor VIII:C activity derived from the full-length factor VIII polypeptide by deleting one or more amino acids. The term "factor VIII:QD" is defined as a polypeptide chain derived from full-length factor VIII lacking amino acids 745 through 1562. The term "factor VIII:RE" is defined as a polypeptide chain derived from full-length factor VIII lacking amino acids 741 through 1648. The term factor VIII:SQ is defined as a polypeptide chain derived from full-length factor VIII lacking amino acids 743 through 1636.

### PRIOR ART

In fresh plasma prepared in the presence of protease inhibitors, factor VIII has been shown to have a molecular weight of 280 kDa and to be composed of two peptide chains with molecular weights of 200 kDa and 80 kDa, respectively (Andersson, L-O et al. (1986) Proc.Natl.Acad.Sci. USA 83, 2979-2983). These chains are held together by metal ion bridges. More or less proteolytically degraded forms of the factor VIII molecule were found as active fragments in factor VIII material purified from commercial concentrates (Andersson, L-O et al. 1986; Andersson, et al. (1985) EP 0197901 ). The fragmented forms of factor VIII having molecular weights from 260 kDa down to 170 kDa, consisted of one heavy chain with a molecular weight ranging from 180 kDa down to 90 kDa (all variants having the identical N-termini) in combination with the 80 kDa light chain. The N-terminal region of the heavy chains is identical to that of the single chain factor VIII obtained from the nucleotide sequence data of the factor VIII cDNA (Wood, W.I., et aL (1984) Nature 312, 330-336, Vehar, G.A., et al., (1984) Nature 312, 337342).

The smallest active form, with molecular weight 170 kDa, containing one 90 kDa and one 80 kDa chain could be activated with thrombin to the same extent as the higher molecular forms. It thus represents an unactivated form. It has also been shown to have full biological activity in vivo as tested in hemophilia dogs (Brinkhous, K.M. et al. (1985) Proc.Natl.Acad.Sci. USA 82, 8752-8756). Thus, the haemostatic effectiveness was the same as for the high molecular weight forms of factor III. Furthermore, there was an indication that the 170 kDa form had an in vivo survival time that was 50% longer as compared to that of the higher molecular forms.

The fact that the middle heavily glycosylated part of the factor VIII polypeptide chain residing between amino acids Arg-739 and Glu-1649 does not seem to be necessary for full biological activity has prompted several researchers to attempt to produce derivatives of recombinant factor VIII lacking this region. This has been achieved by deleting a portion of the cDNA encoding the middle heavily glycosylated part of factor VIII either entirely or partially.

For example, J.J. Toole, et al. reported the construction and expression of factor VIII lacking amino acids 982 through 1562, and 760 through 1639, respectively (Proc.Natl. Acad.Sci. USA (1986) 83, 5939-5942). D.L. Eaton et al. reported the construction and expression of factor VIII lacking amino acids 797 through 1562 (Biochemistry (1986) 25, 8343-8347). R.J. Kaufman described the expression of factor VIII lacking amino acids 741 through 1646 (PCT application No. WO 87/04187). N. Sarver et al. reported the construction and expression of factor VIII lacking amino acids 747 through 1560 (DNA (1987) 6, 553-564). M. Pasek reported the construction and expression of factor VIII lacking amino acids 745 through 1562, and amino acids 741 through 1648, respectively (PCT application No. 88/00831). K-D Langner reported the construction and expression of factor VIII lacking amino acids 816 through 1598, and amino acids 741 through 1689, respectively (Behring Inst. Mitt., (1988) No 82, 16-25, EP 295597). P. Meulien, et al., reported the construction and expression of Factor VIII lacking amino acids 868 through 1562, and amino acids 771 through 1666, respectively (Protein Engineering (1988) 2(4), 301-306, EP 0 303 540 A1). When expressing these deleted forms of factor VIII cDNA in mammalian cells the production level is typically 10 times higher as compared to full-length factor VIII.

Furthermore, attempts have been made to express the 90 kDa and 80 kDa chains separately from two different cDNA derivatives in the same cell (Burke, R.L. et al. (1986), J.Biol.Chem. 261, 12574-12578, Pavirani, A. et al. (1987) Biochem. Biophys. Res. Comm., 145, 234-240). However, in this system the in vivo reconstitution seems to be of limited efficiency in terms of recovered factor VIII:C activity.

### DESCRIPTION OF THE INVENTION

The present invention deals with processes for production of proteins consisting of one or more polypeptide chains having factor VIII:C activity. More specifically, the present invention provides a modified cDNA sequence derived from the full-length factor VIII cDNA, which upon expression in animal cells gives rise to the high level production of a protein with factor VIII:C activity consisting essentially of two polypeptide chains of 90 kDa and 80 kDa molecular weight, respectively.

Accordingly, the present invention provides for a DNA sequence coding fora biologically active recombinant human factor VIII derivative, comprising a first DNA segment coding for the 90 kDa chain of human factor VIII and a second DNA segment coding for the 80 kDa chain of human factor VIII said segment being interconnected by a linker DNA segment coding for a linker peptide of 14 amino acid residues of the B domain of human factor VIII, wherein said linker DNA segment codes for 12 amino acid residues originating from the C terminal and 2 amino acid residues originating from the N terminal of the B domain of human factor VIII.

The invention also relates to a recombinant expression vector containing a transcription unit comprising the DNA sequence as outlined above and, furthermore, a promoter and a polyadenylation signal sequence.

The invention also covers host cells of animal origin transformed with the recombinant expression vector as defined above.

The invention also relates to a process for the manufacture of a biologically active recombinant human factor VIII derivative expressed by a DNA sequence that codes for biologically active recombinant human factor VIII derivative composed of two polypeptide chains of 90 kDa and 80 kDa molecular weight, respectively, said DNA sequence being capable of expressing, in an appropriate host cell, said derivative for secretion from said cell, and comprising a first DNA segment coding for the 90kDa chain of human factor VIII and a second DNA segment coding for the 80 kDa chain of human factor VIII, said segments being interconnected by a linker DNA segment coding for a linker peptide of at least 7 and up to 20 amino acid residues of the B domain of human factor VIII, at least 4 of said amino acid residues originating from the C-terminal of said domain and at least 2 amino acid residues originating from the N-terminal of said domain, the process characterized by
a) cultivating an animal cell line transformed with a recombinant expression vector containing a transcription unit comprising said DNA sequence, a promoter and a polyadenylation signal sequence in a nutrient medium;
b) allowing expression and secretion of a human factor VIII derivative composed of two polypeptides with molecule weights of 90 kDa and 80 kDa, respectively, linked to each other by a metal ion bridge;
c) recovering said derivative from the culture medium; and
d) formulating said derivative into pharmaceutical preparations.

In order to obtain a protein with factor VIII:C activity consisting of the above two polypeptide chains, the single polypeptide chain created during translation in vivo has to be cleaved either by post-translational processing in the producing cell or by proteolytic processing in vitro, or both. Since protein with factor VIII activity, consisting of the two polypeptide chains of 200 kDa and 80 kDa molecular weight, can be isolated from human plasma, It Is assumed that there exists an appropriate cleavage site for processing enzymes, according to the above, on the single chain full-length factor VIII primary translation product. Most probably, an important in vivo processing site is located at the carboxy-terminal side of Arg-1648. During maturation, cleavage at Arg-1648 gives rise to a factor VIII protein consisting of two chains of molecular weights of 200 kDa and 80 kDa. Since Arg-1648 seems to be located at a border between structural domains in the factor VIII molecule, it constitutes a sterically accessible target for the processing enzyme or enzymes. Conceivably, there exists another processing site at Arg-740 which will give rise to conversion of the 200 kDa chain to a 90 kDa chain in vitro, thus creating the 90 kDa and 80 kDa form of factor VIII present in commercial human plasma derived factor VIII concentrates. In accordance with the present invention has been found that in order to produce a factor VIII deletion derivative that can be processed either in vivo or in vitro, or both, to a two chain protein consisting of polypeptide chains of 90 kDa and 80 kDa, the amino acid sequences surrounding Arg-740 and Arg-1648 have to be conserved in order to preserve the structural requirements for correct cleavage. More specifically, it for example amino acids 743 through 1635 of the full-length factorVIII polypeptide are deleted, a new polypeptide chain is obtained where there are 14 amino acids linking Arg-740 and Arg-1648. Of these 14 amino acids, the sequence of the five amino-terminal ones directly corresponds to the five amino acids following Arg-740 in full-length factor VIII. Also, the sequence of the 12 carboxy-terminal amino acids of the above 14 amino acid fragment directly corresponds to the 12 amino acids preceding Glu-1649 in full-length factor VIII thus creating a 3 amino acid overlap between the N- and C-terminal regions of the B-domain.

In order to produce a factor VIII:SQ protein at high level consisting of two polypeptide chains according to the above, the cDNA encoding this factor VIII deletion derivative is assembled into an efficient transcriptional unit together with suitable regulatory elements in a cloning vector, that can be propagated in E.coli according to methods known to those skilled in the art. Efficient transcriptional regulatory elements could be derived from the chromosomal DNA of eucaryotic cells. For instance, promoter-enhancer combinations from strongly constitutively transcribed genes can be used, preferably metaliothionein, beta-actin or GRP7B. Also, efficient transcriptional regulatory elements can be derived from viruses having eukaryotic cells as their natural hosts. For instance, promoter-enhancer combinations derived from viruses residing on animal hosts can be used, preferably the enhancer and promoter occurring in Rous sarcoma virus long terminal repeat, simian virus 40, the adenovirus major late promoter, and the human cytomegalovirus immediate early promoter. In order to obtain stable high level amounts of mRNA transcribed from the factor VIII:SQ cDNA, the transcriptional unit should contain in its 3'-proximal part a DNA region encoding a transcriptional termination-polyadenylation sequence. Preferably, this sequence is derived from the Simian Virus 40 early transcriptional region or the rabbit beta-globin gene.

The factor VIII:SQ cDNA assembled into an efficient transcription unit is then introduced into a suitable host organism for expression of the factor VIII:SQ protein. Preferably this organism should be an animal cell-line of vertebrate origin in order to ensure correct folding, disulfide bond formation and secretion as well as other post-transtational modifications. Examples of the latter are asparagine-linked glycosylation, tyrosine O-sulfatation, and proteolytic processing of the nascent single polypeptide chain which is essential for the formation of the 90 kDa and 80 kDa two chain factor VIII protein. Examples of cell-lines that can be used are moneky COS-caffs, mouse L-cells, mouse C127-cells, hamster BHK-21 cells, and preferentially CHO-cells.

The transcription unit encoding the factor VIII:SQ cDNA can be introduced into an animal cell-line in several different ways. One example of this is to create recombinants between the above transcription unit and vectors based on different animal viruses. Examples of these are vectors based on vaccinia virus, SV40 virus, and preferably bovine papilloma virus.

The transcription unit encoding the factor VIII:SQ cDNA can also be introduced into an animal cell-line together with another recombinant gene which may function as a dominant selectable marker in these cells in order to facilitate the isolation of specific cell clones which have integrated the recombinant DNA into the genome. Examples of this type of dominant selectable marker genes are Tn5 aminoglycoside phosphotransferase (resistance to Geneticin, G418), and puromycin acetyltransferase (resistance to puromycin). The transcription unit encoding such a selectable marker gene can reside either on the same vector as the one encoding the factor VIII:SQ unit. It can also be encoded on a separate vector which is simultaneous introduced and integrated into the genome of the host cell, frequently resulting in tight physical linkage between the different transcription units.

Other types of dominant selectable marker genes which can be used together with the factor VIII:SQ cDNA are based on various transcription units encoding dihydrofolate reductase (dhfr). After introduction of this type of gene into cells lacking endogenous dhfr-activity, preferentially CHO-cells (DUKX-B11, DG-44) it will enable these to grow in medium tacking nucleosides. An example of such a medium is Ham's F12 without hypoxanthin, thymidin, and glycine. These dhfr-genes can be used in conjunction with the factor VIII:SQ transcriptional unit in CHO-cells of the above type, either linked on the same vector or in two different vectors, thus creating dhfr-positive cell-lines producing recombinant factor VIII:SQ protein.

If the above call-lines are grown in the presence of the cytotoxic dhtr-inhibitor methotrexate, new cell-lines resistant to methotrexate will emerge. These cell-lines may produce recombinant factor VIII:SQ protein at an increased rate due to the amplified number of linked dhfr and factor VIII:SQ transcriptional units. When propagating these cell-lines in increasing concentrations of methotrexate (1-10000 nM), new cell-lines can be obtained which produce the factor VIII: SQ protein at very high rate.

The above cell-lines producing factor VIII:SQ protein can be grown in a large scale, either in suspension culture or on various solid supports. Examples of these supports are microcarriers based on dextran or collagen matrices, or solid supports in the form of hollow fibers or various ceramic materials. When grown in suspension culture or on microcarriers the culture of the above cell-lines can be performed either as a batch culture or as a perfusion culture with continuous production of conditioned medium over extended periods of time. Thus, according to the present invention, the above cell-lines are well suited for the development of an industrial process for the production of recombinant factor VIII:SQ which corresponds to the authentic two polypeptide chain factor VIII (90 kDa and 80 kDa) that can be isolated from human plasma.

The recombinant factor VIII:SQ protein which accumulates in the medium of CHO-cells of the above type, can be concentrated and purified by a variety of biochemical methods, including methods utilizing differences in size, charge, solubility, hydrophobicity, specific affinity, etc. between the recombinant factor VIII:SQ and other substances in the conditioned medium.

An example of such a purification is the adsorption of the recombinant factor VIII:SQ protein to a monoclonal antibody which is immobilised on a solid support. After desorption, the factor VIII:SQ protein can be further purified by a variety of chromatographic techniques based on the above properties.

The protein with factor VIII activity described in this invention can be formulated into pharmaceutical preparations for therapeutic use. The produced recombinant factor VIII:SQ protein may be dissolved in conventional physiologically compatible aqueous buffer solutions to which there may be added, optionally, pharmaceutical adjuvants to provide pharmaceutical preparations.

The present invention will be further described more in detail in the following by non-limiting examples thereof. This description of specific embodiments of the invention will be made in conjunction with the appended drawings, wherein:

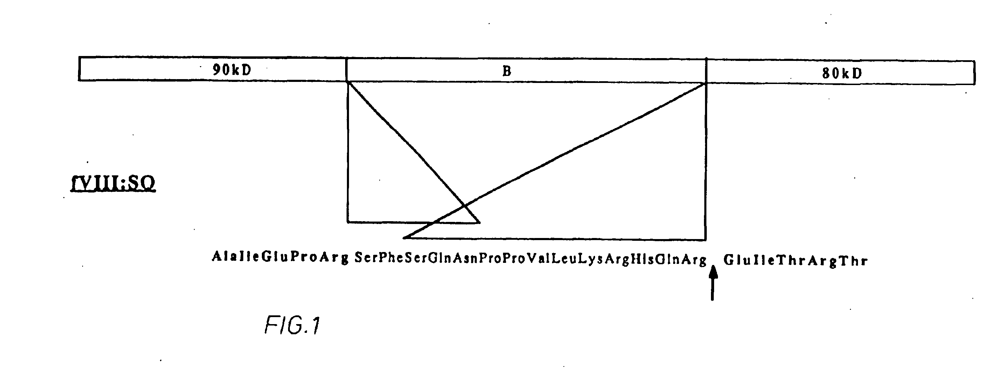

Figure 1 is a schematic representation of the relationship between full-length factor VIII and factor VIII:SQ. The primary structure of the region between the C-terminus of the 90 kDa chain and the N-terminus of the 80 kDa chain is shown. The vertical arrow indicates the peptide bond that is cleaved to give the two-chain product;
Figure 2 is an illustration of plasmid pKGE436 containing the factor VIII:SQ cDNA under transcriptional control of the human cytomegalovirus promoter,
Figure 3 is an illustration of plasmid pKGE327 containing the mouse dihydrofolate reductase cDNA under transcriptional control of the mouse mammary tumor virus long terminal repeat;
Figure 4 is an illustration of immuno-blotting of recombinant factor VIII SQ after polyacrylamide gel electrophoresis in the presence of sodium dodecyl sulfate;
Figure 5 shows a diagram on changes in factor VIII activity of recombinant factor VIII:SQ following incubation with thrombin: and
Figure 6 Illustrates changes in patterns of SDS-PAGE and immunoblotting of recombinant factor VIII:SQ following incubation with human α-thrombin.

### EXAMPLE 1

A deletion derivative of factor VIII cDNA has been constructed that encodes a polypeptide chain devoid of most of the B-domain, but containing parts of the amino-terminal and carboxy-terminal sequences of the B-domain that are essential for in vivo proteolytic processing of the primary translation product into two polypeptide chains.

### Mutagenesis of factor VIII cDNA.

A 894 base-pair Kpnl-Pstl restriction fragment obtained from the cDNA of the factor VIII:QD deletion derivative (M. Pasek, PCT application No. WO88/00831) encoding the amino acids Leu 587 through Ata 1702 was introduced into the bacteriophage vector M13mp19 (Yanisch-Perron, C. et al. (1985) Gene 33, 103-119) according to the standard methods of the art. The resulting recombinant phage clone was used as source for preparation of single stranded DNA used as template for oligonucleotide directed mutagenesis (Nakamaye, K. and Eck-stain, F. (1986) Nucleic Acids Res. 14, 9679-9698). 10µg purified circular single stranded vector DNA was annealed to 8 pmoles of a 5'-phosphorylated oligonucleotide with the following sequence:

5'-GCCATTGAACCAAGAAGCTTCTCTCAGAATCCACCAGTCTTGAAACGC-3'

The second strand of circular DNA was synthesised on the resulting template by addition of all tour deoxynucleotides, dCTPαS, 12 units of Klenow fragment of DNA-polymerase I, and 12 units of T4 DNA-ligase. After overnight incubation at 16°C, the reaction mixture was enriched for double stranded DNA by filtering through nitrocellulose In the presence of 500 mM NaCL One fifth of the purified double stranded DNA was nicked by incubation with 5 units of the restriction enzyme Ncil, treated by 50 units of Exonuclease III to such an extent that the template strand of the phage DNA was partially removed. The resulting partial duplex was reconverted to double stranded DNA by treatment with 3 units DNA polymerase I and 2 units T4 DNA-ligase in the presence of all four deoxynucleotide triphosphates at 16°C for 3 hours. One fourth of the resulting mixture was used to transform 300 µl E.coli TG1. Of the resulting muta-genised phage clones, ten were subjected to dideoxy sequencing (Sanger, F. et al. 1977, Proc. Natl.Acad.Sci. USA 74, 5463-5467). One of the resulting phage clones showed the expected nucleotide sequence indicating that a 228 base-pair segment had been removed which corresponds to amino acids Asp 1563 through Ser 1637 of factor VIII: QD. This creates a novel 666 base-pair Kpnl -Pstl fragment of the factor VIII cDNA within the M13mp19 vector encoding a fusion between Phe 742 and Ser 1637 of the factor VIII protein (factor VIII:SQ).

### Construction of a Mammalian Expression Vector Encoding factor VIII:SQ.

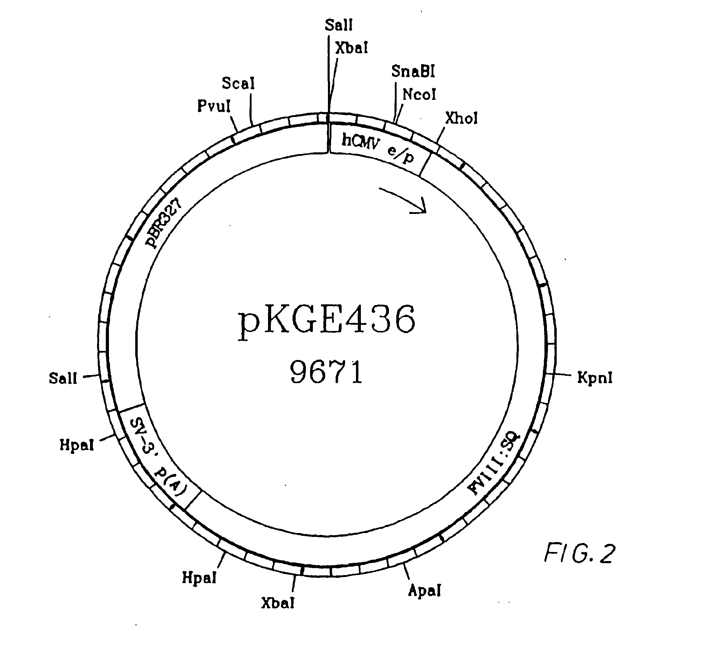

The 666 base-pair Kpnl-Pstl fragment encoding the factor VIII:SQ fusion according to the above was isolated from the double stranded replicative form of the M13mp19 phage DNA and introduced into the vector pKGE431. This vector consists of a 2046 base-pair Kpnl-Sphl fragment from the factor VIII:RE cDNA (M. Pasek, PCT application No. WO88/00831, ATCC accession No. 53517) encoding amino acids Leu 587 through Met 2176 of the factor VIII:RE protein in pUC19. In order to accommodate the factor VIII:SQ 666 base-pair Kpnl-Pstl fragment in a desired manner the pKGE431 DNA had to be opened by complete Kpnl digestion and partial Pstl digestion. The fragment of pKGE431 in which Pstl cleaved in a position corresponding to Ala 1702 in the factor VIII:RE protein was isolated and ligated to the above Kpnl-Pstl fragment of the factor VIII:SQ cDNA creating the vector pKGE432. The latter vector was digested with Kpnl and Apal and the corresponding 1700 base-pair Kpnl-Apal fragment encoding Leu 587 through Ala 2047 of the factor VIII:SQ protein was ligated to the large fragment of the vector pKGE347 that had been digested with Kpnl and Apal. The vector pKGE347, which is based on the E.coli cloning vector pBR327, consists of the human cytomegalovirus enhancer/promoter encoded on a 741 base-pair DNA-segment (nucleotide positions - 671 to +71, Boshart, M. et al (1985) Cell 41, 521-530) upstream of the factor VIII:OD cDNA with the SV40 t-antigen intron and polyadenylation sequence at the 3'-proximal part. The resulting vector (pKGE436), which is depicted in Figure 2, contains the complete factor VIII:SQ cDNAand is identical to pKGE347 except for the different deletion derivative of factor VIII that is encoded.

### EXAMPLE 2

### Transfection of Chinese Hamster Ovary Cells.

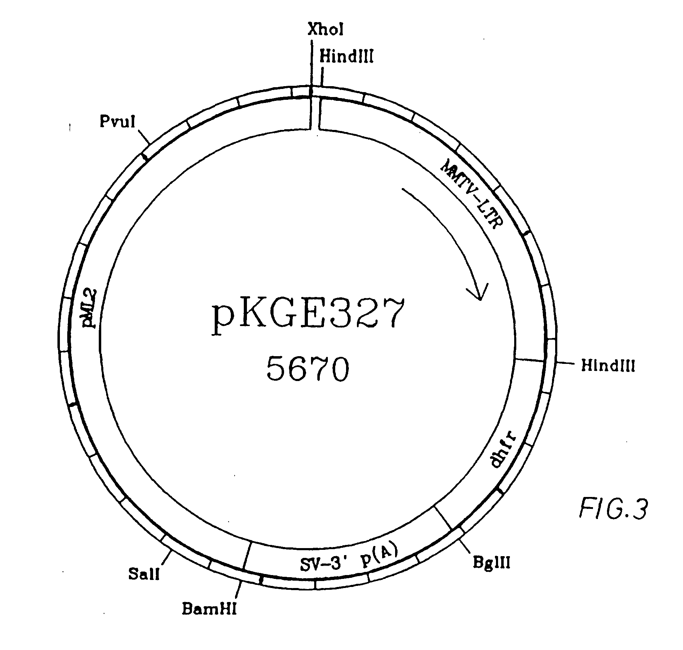

In a 10 centimeter diameter cell culture dish, 0.5 million dihydrofolate reductase deficient Chinese Hamster Ovary Cells (CHO-DG44, obtained from Dr. LA. Chasin, Columbia University. New York) were seeded in Dulbacco's Modified Eagles Medium/Ham's F12 (1:1) supplemented by 10% foetal call serum and incubated over night at 37°C in a 5% carbon dioxide incubator. The next day the cells were washed with fresh medium and subsequently transfected by the calcium phosphate method with t0 µg of a 1:1 mixture of the factor VIII:SQ expression vector pKGE435 and the dihydrofolate reductase vector pKGE327 according to the methods of the art. The vector pKGE327 contains a transcription unit consisting of the mouse mammary tumor virus long terminal repeat upstream of the mouse dihydrofolate reductase cDNA with the SV40 t-antigen and polyadenylation sequence at the 3'-proximal part cloned into the vector pML2 in a clockwise manner (Figure 3). On day three, the medium was removed, the cells washed and split into new cell cultivation dishes. On day four, the selection for dihydrofolate reductase positive cells was initiated by replacing the media with the above cell cultivation medium lacking hypoxanthin, glycin, and thymidin and supplemented with 10% thoroughly dialyzed foetal calf serum. The medium was changed twice a week and after approximately two weeks colonies of dihydrofolate reductase positive cells could be harvested. These colonies were further grown in 25 cm² cell culture bottles, and after reaching subconfluency the medium was replaced with fresh medium containing 3X foetal calf serum. After a period of 24 hours the activity of the factor VIII:C in the culture medium was tested using the synthetic substrate method (Coatest factor VIII:C, KABI). The results are shown below in Table 1.

**TABLE 1**

| CHO-DG44 cell-lines producing FVIII SQ | | | |
|---|---|---|---|
| Cell-line No | Coatest assay (mU/ml) | Cell-line No | Coatest assay (mU/ml) |
| 208:1 | 10 | 208:13 | 107 |
| 2 | 73 | 14 | 167 |
| 3 | 147 | 15 | 253 |
| 4 | 27 | 16 | 253 |
| 5 | 107 | 17 | 313 |
| 6 | 80 | 18 | 307 |
| 7 | 73 | 19 | 27 |
| 8 | 87 | 20 | 287 |
| 9 | 93 | 21 | 393 |
| 10 | 33 | 22 | 467 |
| 11 | 47 | 23 | 107 |
| 12 | 147 | 24 | 180 |

### Gene Amplification of CHO-DG44 Cell Lines Producing factor VIII:SQ

The CNO-DG44 cell line 203:22 in Table 1 producing 467 mU/ml/day of factor VIII:SQ was chosen for further gene amplification by selection and growth in methotrexate. After several weeks of cultivation in 20 nM methotrexate the call line 208:22 gave rise to several new resistant cell clones. These clones were harvested individually and further expanded as separate cultures as described above. The productivity of factor VIII:C in the medium of these clones were assayed as in Example 1. The result is shown in Table 2.

**TABLE 2**

| Amplified cell-lines producing factor VIII:SQ derived from the CHO-DG44 cell-line 208:22 | | | |
|---|---|---|---|
| Cell-line No. | Coatest assay (mU/ml) | Cell-line No. | Coatest assay (mU/ml) |
| 208:22:1 | 1040 | 208:22:13 | 1070 |
| 2 | 690 | 14 | 860 |
| 3 | 660 | 15 | 930 |
| 4 | 720 | 16 | 680 |
| 5 | 800 | 17 | 790 |
| 6 | 940 | 18 | 750 |
| 7 | 1100 | 19 | 960 |
| 8 | 260 | 20 | 80 |
| 9 | 900 | 21 | 1130 |
| 10 | 660 | 22 | 920 |
| 11 | 720 | 23 | 950 |
| 12 | 870 | 24 | 1010 |

### EXAMPLE 3

### Purification and Biochemical Characterization of factor VIII:SQ

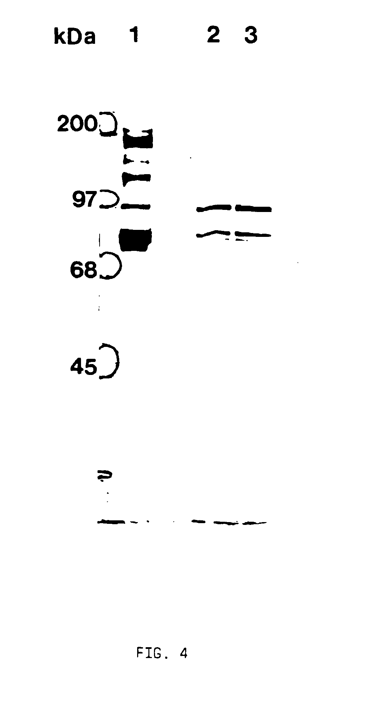

Factor VIII:SQ produced by the amplified CHO-DG44 cell-line 208:22 was examined for biochemical characteristics. A partial purification of the factor VIII material from culture medium was performed prior to the study. This included an immunoaffinity chromatography step with the use of a matrix containing monoclonal antibodies directed against factor VIII followed by an ion-exchange chromatography step. The specific activity of the purified factor VIII material obtained was in the range of 3000-4000 IU/A₂₈₀ and the ratio factor VIII activity/factor VIII antigen was close to 1 (activity was measured with Coatest (KABI), and antigen was determined by an Elisa assay with use of monoclonal antibodies directed against the 80 kDa chain). The purified factor VIII:SQ material was submitted to SDS-polyacrylamide gal electrophoresis (SDS-PAGE) and Western blot analysis. The SDS-PAGE was carried out according to Laemmli (1970) Nature 227, 680-685. Rabitt polyclonal anti-human factor VIII antibodies were used for the Western blot analysis and the analysis was essentially performed as described by Towbin, H. at al. (1979) Proc. Natl.Acad.Sci. USA, 76, 4350-4354. The results obtained are shown in Fig. 4, Lane 1: plasma factor VIII containing an 80 kDa light chain and heavy chains ranging from 200 kDa to 90 kDa. Lanes 2 and 3: recombinant factor VIII:SQ containing an 80 kDa chain and a 90 kDa chain. The materials originated from two different batches factor VIII:SQ purified as described above. The analysis thus showed the presence of two major bands in the factor VIII SQ material; one at 90 kDa and one doublet band at 80 kDa. The two bands were found in the same position on the gel as the 90 kDa and 80 kDa peptides representing the smallest biologically active complex of plasma factor VIII. A minor amount of a 170 kDa band (< of 5X of the total material) was also found in the factor VIII:SQ material, probably representing uncleaved primary translation product. Furthermore, N-terminal sequence determination with automated Edman degradation of the factor VIII: SQ protein, indicated that the N-termini of the 90 kDa and 80 kDa chains are identical to those of plasma derived 90 kDa plus 80 kDa factor VIII. This result thus showed that the in vivo proteolytic processing of the primary translation product into two polypeptide chains had been effective.

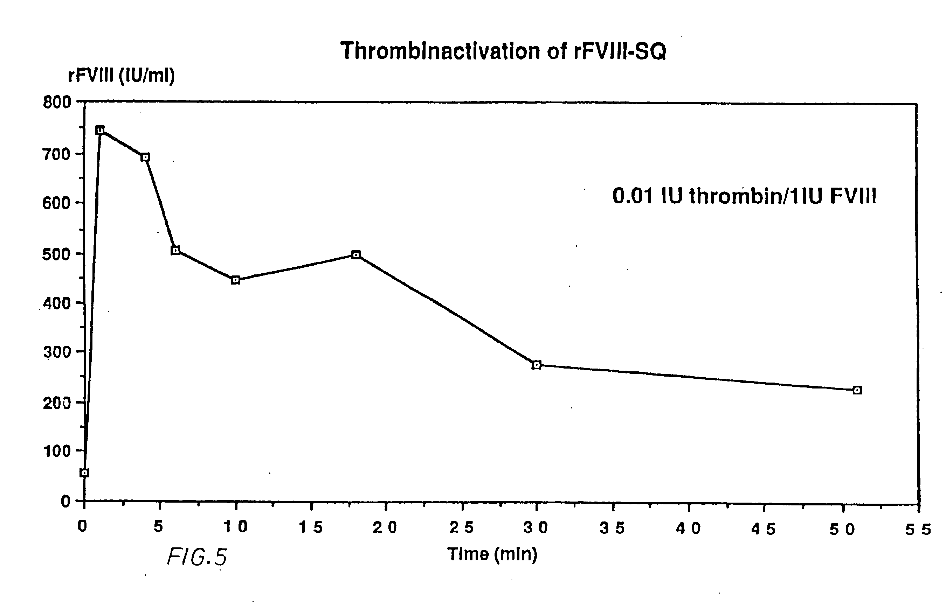

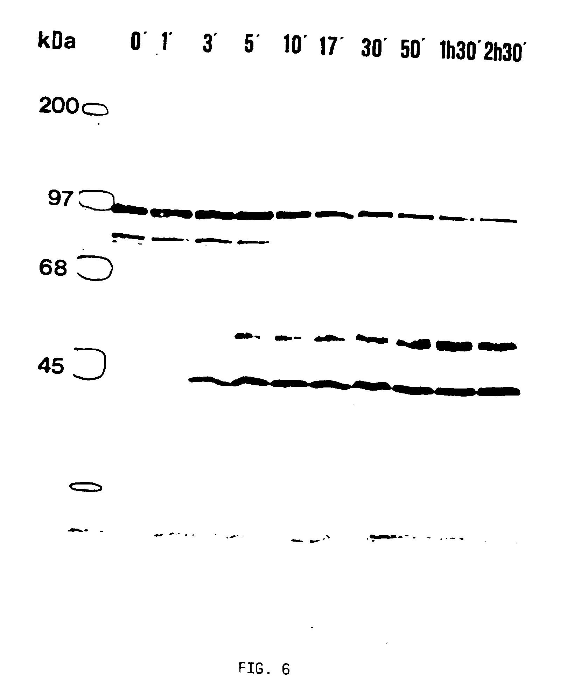

Factor VIII:SQ could be activated by human thrombin In a dose dependent manner Inactivation was subsequently followed. In Figure 5, the curve of activity changes obtained is shown when 0.1 U thrombin per 1 IU purified factor VIII: SQ was added. A one-stage clotting method (Mikaelsson, M. et al. (1983) Blood 62, 1006-1015) was used for immediate assay of samples from the reaction mixture. A fifteen-fold activation was obtained whithin one minute, which then was followed by inactivation. Sodium dodecyl sulfate at 0.02 g/ml was added to stop the reaction in samples for analysis. SDS-Polyacrylamide gel electrophoresis and Wastern blot analysis with rabbit polyclonal antibodies against human factor VIII was performed on samples taken out at time intervals during the reaction (Figure 6). Electrophoresis and immunoblotting were performed as described in relation to Figure 4.

The results obtained showed a molecular change of factor VIII peptides identical to that of plasma factor VIII during incubation with thrombin. Thus the 90 kDa peptide was cleaved by thrombin and a 50 kDa plus a 40 kDa peptide was formed. The 80 kDa peptide was cleaved and a 70 kDa peptide was formed. The weak band seen at 170 kDa in the factor VIII:SQ sample disappeared during the first minute of incubation with thrombin, and peptides were apparently formed identical to those formed from the 90 kDa-80 kDa complex. The studies with thrombin showed that factor VIII: SQ behaves as plasma factor VIII in the interaction with this enzyme. This feature is regarded to be essential for biological activity in vivo.

The interaction of factor VIII:SQ with human von Willebrand factor was studied with the use of size-exclusion chromatography on Sepharose CL-6B. Ten (10)IU of factor VIII:SO was incubated with 30 U purified human von Willebrand factor at 37°C for 20 minutes. The incubation mixture was then applied on a column packed with Sepharose CL-6B. All material with factor VIII activity eluted in the void volume with von Willebrand factor. When factor VIII:SQ with no added von Willebrand factor was applied on the column, material with factor VIII activity eluted only in the inner fractions at a position where also the 90 kDa-80 kDa form of plasma factor VIII eluted. This result shows that factor VIII:SQ has the capacity to bind to von Willebrand factor, which is a property necessary for good in vivo survival (Brinkhous, KM. et al (1985) Proc.Natl.Acad.Sci. USA 82, 8752-8756).

The factor VIII:SQ DNA of the present invention has been deposited with Deutsche Sammlung von Mikroorganisman und Zellkulturen on December 13, 1939 and has been given the deposition number DSM 5693.

## Claims

1. A DNA sequence coding for a biologically active recombinant human factor VIII derivative composed of two polypeptide chains of 90 kDa and 80 kDa molecular weight, respectively, said DNA sequence being capable of expressing, in an appropriate host cell, said derivative for secretion from said cell, and comprising a first DNA segment coding for the 90kDa, chain of human factor VIII and a second DNA segment coding for the 80 kDa chain of human factor VIII, said segments being interconnected by a linker DNA segment coding for a linker peptide of 14 amino acid residues of the B domain of human factor VIII, wherein said linker DNA segment codes for 12 amino acid residues originating from the C-terminal and 2 amino acid residues originating from the N-terminal of the B domain of human factor VIII.

2. A DNA sequence according to claim 1, wherein said linker DNA segment codes for a linker consisting of the sequence Ser-Phe-Ser-Gln-Asn-Pro-Pro-Val-Leu-Lys-Arg-His-Gln-Arg.

3. A recombinant expression vector containing a transcription unit comprising the DNA sequence according to any one of claims 1 to 2, a promoter and a polyadenylation signal sequence.

4. A host cell of animal origin transformed with the recombinant expression vector of claim 3.

5. A process for the manufacture of a biologically active recombinant human factor VIII derivative expressed by a DNA sequence that codes for biologically active recombinant human factor VIII derivative composed of two polypeptide chains of 90 kDa and 80 kDa molecular weight, respectively, said DNA sequence being capable of expressing, in an appropriate host cell, said derivative for secretion from said cell, and comprising a first DNA segment coding for the 90kDa chain of human factor VIII and a second DNA segment coding for the 80 kDa chain of human factor VIII, said segments being interconnected by a linker DNA segment coding for a linker peptide of at least 7 and up to 20 amino acid residues of the B domain of human factor VIII, at least 4 of said amino acid residues originating from the C-terminal of said domain and at least 2 amino acid residues originating from the N-terminal of said domain, the process **characterized by**
a) cultivating an animal cell line transformed with a recombinant expression vector containing a transcription unit comprising said DNA sequence, a promoter and a polyadenylation signal sequence in a nutrient medium;
b) allowing expression and secretion of a human factor VIII derivative composed of two polypeptides with molecule weights of 90 kDa and 80 kDa, respectively, linked to each other by a metal ion bridge,
c) recovering said derivative from the culture medium; and
d) formulating said derivative into pharmaceutical preparations.

## Patentansprüche

1. DNA-Sequenz, die ein biologisch aktives, rekombinantes Derivat von menschlichem Faktor VIII, das aus zwei Polypeptidketten mit Molekulargewichten von 90 kDa bzw. 80 kDa besteht, kodiert, wobei die DNA-Sequenz für eine Expression des Derivates in einer geeigneten Wirtszelle zur Absonderung aus dieser Zelle geeignet ist and einen ersten DNA-Abschnitt, der die 90 kDa schwere Kette von menschlichem Faktor VIII kodiert, and einen zweiten DNA-Abschnitt, der die 80 kDa schwere Kette von menschlichem Faktor VIII kodiert, umfaßt und diese Abschnitte durch einen Verbindungs-DNA-Abschnitt, der ein Verbindungspeptid von 14 Aminosäureresten aus der B-Domäne von menschlichem Faktor VIII kodiert, wobei besagter Verbindungs-DNA-Abschnitt eine Aminosäuresequenz mit 12 Aminosäuren, die aus dem C-Terminus und 2 Aminosäuren, die aus dem N-Terminus der B-Domäne von menschlichem Faktor VIII stammen, kodiert.

2. DNA-Sequenz nach Anspruch 1, wobei besagter Verbindungs-DNA-Abschnitt ein Verbindingspeptid kodiert, bestehend aus der Sequenz Ser-Phe-Ser-Gln-Asn-Pro-Pro-Val-Leu-Lys-Arg-His-Gln-Arg.

3. Rekombinanter Expressionsvektor, der eine Transkriptionseinheit enthält, welche die DNA-Sequenz nach einem der Ansprüche 1 bis 2, einen Promotor und eine Polyadenylierungssignalsequenz umfaßt.

4. Wirtszelle tierischen Ursprungs, die mit dem rekombinanten Expressionsvektor nach Anspruch 3 transformiert ist.

5. Verfahren zur Herstellung eines biologisch aktiven, rekombinanten Derivates von menschlichem Faktor VIII, das von einer DNA-Sequenz die ein biologisch aktives, rekombinantes Derivat von menschlichem Faktor VIII, das aus zwei Polypeptidketten mit Molekulargewichten von 90 kDa bzw. 80 kDa besteht, kodiert, wobei die DNA-Sequenz für eine Expression des Derivates in einer geeigneten Wirtszelle zur Absonderung aus dieser Zelle geeignet ist and einen ersten DNA-Abschnitt, der die 90 kDa schwere Kette von menschlichem Faktor VIII kodiert, and einen zweiten DNA-Abschnitt, der die 80 kDa schwere Kette von menschlichem Faktor VIII kodiert, umfaßt und diese Abschnitte durch einen Verbindungs-DNA-Abschnitt, der ein Verbindungspeptid von mindestens 7 und bis zu 20 Aminosäureresten aus der B-Domäne von menschlichem Faktor VIII kodiert, wobei wenigstens 4 dieser Aminosäurereste aus dem C-Terminus dieser Domäne stammen und 2 Aminosäuren, aus dem N-Terminus dieser Domäne stammen, das Verfahren **gekennzeichnet durch**:
a) Kultivieren einer tierischen Zelllinie, die mit einem rekombinanten Expressionsvektor, der eine Transkriptionseinheit, die besagte DNA Sequenz, einen Promoter und eine Polyadenylierungssignalsequenz umfaßt, transformiert ist, in einem Nährmedium;
b) Erlauben der Expression und Absonderung eines Derivates von menschlichem Faktor VIII, das aus zwei Polypeptidketten mit Molekulargewichten von 90 kDa bzw 80 kDa besteht, die über eine Metallionenbrücke miteinander verbunden sind;
c) Gewinnung des Derivates aus dem Kulturmedium; und
d) Formulierung besagten Derivates in pharmazeutischen Präparaten.

## Revendications

1. Séquence d'ADN codant pour un dérivé de facteur VIII humain recombinant biologiquement actif, composé de deux chaînes polypeptidiques ayant respectivement un poids moléculaire de 90 kDa et 80 kDa, ladite séquence d'ADN étant capable d'exprimer, clans une cellule hôte appropriée, ledit dérivé pour une sécrétion à partir de ladite cellule, et comprenant un premier segment d'ADN codant pour la chaîne de 90 kDa du facteur VIII humain et un second segment d'ADN codant pour la chaîne de 80 kDa du facteur VIII humain, lesdits segments étant reliés entre eux par l'intermédiaire d'un segment d'ADN lieur codant pour un peptide de liaison ayant jusqu'à 14 résidus d'acides aminés du domaine B du facteur VIII humain, dans laquelle ledit segment d'ADN lieur code pour une séquence d'acides aminés comprenant 12 acides aminés issus de la région C-terminale et au moins 2 résidus d'acides aminés étant issus de la région N-terminale du domaine B du facteur VIII humain.

2. Sequence d'ADN selon la revendication 1, dans laquelle ledit segment d'ADN lieur code pour pour un lieur ayant la séquence Ser-Phe-Ser-Gln-Asn-Pro-Pro-Val-Leu-Lys-Arg-His-Gln-Arg.

3. Vecteur d'expression recombinant contenant une unité de transcription comprenant la séquence d'ADN selon une quelconque des revendications 1 à 2, un promoteur et une séquence signal de polyadénylation.

4. Cellule hôte d'origine animale transformée avec le vecteur d'expression recombinant de la revendication 3.

5. Procédé pour la fabrication d'un dérivé de facteur VIII humain recombinant biologiquement actif, exprimé par une séquence d'ADN qui code pour un dérivé de facteur VIII humain recombinant biologiquement actif, composé de deux chaînes polypeptidiques ayant respectivement un poids moléculaire de 90 kDa et 80 kDa, ladite séquence d'ADN etant capable d'exprimer, clans une cellule hôte appropriée, ledit dérivé pour une sécrétion à partir de ladite cellule, et comprenant un premier segment d'ADN codant pour la chaîne de 90 kDa du facteur VIII humain et un second segment d'ADN codant pour la chaîne de 80 kDa du facteur VIII humain, lesdits segments étant reliés entre eux par l'intermédiaire d'un segment d'ADN lieur codant pour un peptide de liaison de au moins 7 et jusqu'à 20 résidus d'acides aminés du domaine B du facteur VIII humain, au moins 4 desdits résidus d'acides aminés étant issus de la région C-terminale dudit domaine et au moins 2 résidus d'acides aminés étant issus de la région N-terminale dudit domaine, le procédé etant **caracterisé par**
a) la mise en culture d'une lignée cellulaire animale transformée avec un vecteur d'expression recombinant contenant une unité de transcription comprenant ledit séquence d'ADN, un promoteur et une séquence signal de polyadénylation dans un milieu nutritive,
b) permettant l'expression et la sécrétion d'un dérivé de facteur VIII humain composé de deux polypeptides ayant respectivement un poids moléculaire de 90 kDa et 80 kDa, liés l'un à l'autre par un pont d'ion métallique,
c) la récupération dudit dérivé à partir du milieu de culture, et
d) formulation dudit dérivé dans préparations pharmaceutiques.
